(19) Europäisches Patentamt / European Patent Office / Office européen des brevets

(11) **EP 3 625 385 B1**

(12) **FASCICULE DE BREVET EUROPEEN**

(45) Date de publication et mention de la délivrance du brevet:
**11.08.2021   Bulletin 2021/32**

(21) Numéro de dépôt: **18730837.4**

(22) Date de dépôt: **17.05.2018**

(51) Int Cl.:
**D04B 21/18** *(2006.01)*

(86) Numéro de dépôt international:
**PCT/FR2018/051191**

(87) Numéro de publication internationale:
**WO 2018/211223 (22.11.2018 Gazette 2018/47)**

(54) **BANDE DE CONTENTION OPTIMISÉE**

OPTIMIERTE KOMPRESSIONSBANDAGE

OPTIMISED COMPRESSION BANDAGE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.05.2017   FR 1754479**

(43) Date de publication de la demande:
**25.03.2020   Bulletin 2020/13**

(73) Titulaire: **Urgo Recherche Innovation et Développement**
**21300 Chenove (FR)**

(72) Inventeurs:
• **COHADE, Céline**
**42560 Saint-Jean-Bonnefonds (FR)**
• **GRANGE, David**
**42210 Bellegarde en Forez (FR)**
• **ROBLOT, Magali**
**21160 Perrigny-les-Dijon (FR)**
• **LECOMTE, Serge**
**21000 Dijon (FR)**

(74) Mandataire: **Cabinet Beau de Loménie**
**158, rue de l'Université**
**75340 Paris Cedex 07 (FR)**

(56) Documents cités:
**EP-A1- 1 052 319     WO-A1-95/16416**

**EP 3 625 385 B1**

**Description**

DOMAINE TECHNIQUE GENERAL

La présente invention est relative à une bande de contention optimisée, dont l'allongement longitudinal est compris entre 30% et 160%, qui est un tricot 3D dont le fil d'entretoise est un multi filaments, obtenu selon la technologie « maille jetée », sans latex ni adhésif, qui ne se détend pas ce qui permet de conserver son efficacité thérapeutique et d'éviter son glissement au cours du temps.

ETAT DE L'ART

L'utilisation de divers systèmes de contention est connue pour traiter les pathologies d'origine veineuse, comme par exemple l'insuffisance veineuse, le traitement des varices et des ulcères de jambes ou encore pour prévenir la thrombose veineuse ou le traitement des lymphoedèmes. Ces systèmes sont constitués d'une ou de plusieurs bandes qui appliquent une pression sur le membre à traiter.
Pour être efficace ce système doit permettre d'appliquer simultanément :

- d'une part, une pression relativement faible appelée « pression de repos », quand le muscle est relâché pour être conformable et en particulier supportable durant la nuit ; et
- d'autre part, une pression relativement élevée appelée « pression de travail », quand le muscle est tendu ou lors des mouvements, en particulier pendant la marche.

Ce différentiel de pression entre pression de travail et pression de repos doit être suffisant pour favoriser le reflux veineux. On considère généralement qu'un différentiel de pression à 24 heures compris entre 15 et 25 mm de mercure est nécessaire pour rétablir un flux veineux correct. Toutefois selon la pathologie, qu'il s'agisse d'un traitement sur des jambes sans ulcères graves, d'un traitement difficile sur des jambes abimées par un œdème, ou d'un traitement d'un ulcère mixte artériel et veineux, cette plage de valeurs peut s'étendre de 10 à 35 mm de mercure voire même de 10 à 40 mm de mercure.
Les bandes de contention utilisées sont classées par les spécialistes de la contention en deux grandes catégories selon la mesure de leur allongement ; les bandes dites à allongement courts et celles dites bandes à allongement long.
Cette classification est basée sur la mesure de l'allongement longitudinal de la bande telle que définie dans la méthode A §9.1 de la norme EN 14704-1 quand la bande est soumise à une force de traction maximale de 6 N/cm.
Les conditions de réalisation de la mesure sont les suivantes.
Une éprouvette du matériau à tester de 50 mm de largueur et de 250 à 300 mm de longueur est découpée et positionnée sans précontrainte dans les mors d'un dynamomètre électronique (par exemple un dynamomètre de marque MTS) de sorte à avoir une largeur de 50 mm et une longueur utile de référence de 200mm. Le dynamomètre étire l'éprouvette à une vitesse de 100 mm/min jusqu'à une force maximale de 6 N/cm puis la traverse revient à sa position initiale à la même vitesse de retour de 100 mm/min. Ce cycle est effectué 5 fois et l'allongement obtenu au cinquième cycle, exprimé en pourcentage, est directement calculé par l'appareillage. L'opération est répétée sur 5 éprouvettes, puis on calcule la valeur moyenne qui définit l'allongement longitudinal de la bande.
L'allongement transversal de la bande peut être évalué selon le même protocole.

Les bandes à allongement court

Sur la base de ce test selon la norme EN 14704-1 pris comme référence, on considère qu'une bande de contention est une bande à «allongement court» si son allongement longitudinal est inférieur ou égal à 100%.
Ces bandes exercent une pression au repos basse et une pression de travail élevée. Elles ont donc un grand différentiel de pression en particulier lors des mouvements, par exemple pendant la marche.

Les bandes à allongement long

Sur la base du test précédent selon la norme EN 14704-1 pris comme référence, on considère qu'une bande est une bande à «allongement long» si son allongement longitudinal est supérieur à 100%.
Ces bandes sont plus faciles à poser car elles présentent une plus grande extensibilité.
Les bandes à allongement long conduisent à de faibles variations de pression entre repos et travail, et à une faible variation de pression au cours des mouvements, par exemple lors de la marche. Elles s'avèrent moins efficaces que les bandes à allongement court. En revanche du fait de ce faible différentiel de pression elles sont soumises lors des mouvements à des contraintes de force plus faibles que les bandes à allongements court, et elles présentent donc un risque de se détendre et donc de glisser le long de la jambe plus faible que les bandes à allongement court.

Il est reconnu aujourd'hui que les systèmes de contention les plus performants en termes de facilité et de rapidité de pose et en termes d'efficacité thérapeutique sont ceux qui comprennent au maximum 2 bandes et au moins une bande de contention dite à allongement court.

A titre d'exemple on peut citer les produits commercialisés sous les dénominations ACTICO, K2 et Coban 2 respectivement par les sociétés ACTIVA, Laboratoires URGO et 3M.

Le système ACTICO est constitué d'une bande à allongement court auto adhérente qui est enroulée sur une bande de ouate préalablement enroulée sur la jambe. La ouate est destinée à répartir les pressions à la surface du membre, et/ou à protéger par son épaisseur les saillies osseuses, et à absorber les éventuels exsudats si la bande est posée sur une plaie ouverte, par exemple dans le cas des ulcères de jambes.

Le système K2® commercialisé par la société Laboratoires URGO est constitué d'une première bande (commercialisée sous la dénomination Ktech®) qui est une bande à allongement court constituée d'une couche d'ouate venant au contact de la peau et aiguilletée à un tricot élastique et d'une seconde bande (commercialisée sous la dénomination KPress®) élastique et auto adhérente qui est une bande à allongement long qui sert à maintenir la première bande en place et à appliquer la pression complémentaire par rapport à la première bande pour obtenir la pression recherchée.

Le système Coban 2 est constitué d'une première bande posée sans extension qui est formée de l'association d'une mousse venant au contact de la peau associée à une bande auto adhérente et d'une seconde bande auto adhérente qui est une bande à allongement court qui applique la pression recherchée et sert à maintenir le système.

Un inconvénient de ces différents systèmes est que pour garantir leur maintien et leur efficacité l'auto adhérence des bandes est obtenue à l'aide d'adhésif ou de latex ce qui complique leur mise au point et peut entraîner des risques d'allergie au contact de la peau, en particulier dans le cas du latex de caoutchouc naturel.

Le rôle de l'adhésif ou du latex est cependant incontournable car c'est lui qui permet de maintenir la bande ou le système après son enroulement autour d'un membre et de diminuer leur relâchement intrinsèque qui conduit à leur perte d'efficacité et leur glissement au cours du temps le long du membre.

En revanche l'incorporation de l'adhésif ou du latex complexifie la réalisation des produits car il modifie les propriétés de pression et de différentiel de pression de la bande sur laquelle il est appliqué.

Afin d'améliorer l'acceptabilité par les patients et le personnel soignant et d'obtenir un produit plus facile à fabriquer il apparait donc souhaitable de disposer d'un système de contention qui utilise des bandes sans adhésif ou latex.

Les tricots dits 3D sont des produits qui se présentent sous la forme de 2 surfaces textiles (des tricots) indépendantes reliées entre elles par des fils d'entretoises d'où leur nom de « 3D ». De tels produits sont par exemple utilisés dans le domaine des sièges automobiles pour leur capacité de compression. Mais pour obtenir cette capacité ces tricots sont épais, rigides et très élastiques. De même ils sont utilisés dans le domaine textile par exemple pour les bonnets de soutien- gorge. Ces tricots sont très doux au toucher mais à nouveau très élastique pour assurer le maintien. Dans les 2 cas ils ne sont pas adaptés pour remplir les propriétés d'une bande de contention.

D'autres tricots 3D adaptés pour la contention ont été proposés dans la demande de brevet WO 95 / 16416.

Le problème que propose de résoudre cette demande WO 95 /16416 est la suppression de la ouate. En effet le tassement de cette dernière au cours du temps induit un jeu entre la jambe et la bande lors des mouvements ce qui peut provoquer le glissement de l'ensemble. L'objectif est de compenser ce phénomène de tassement grâce à la structure 3D et à l'épaisseur du tricot qui permettent d'obtenir un bon effet amortisseur (padding) et de supprimer la ouate. Pour atteindre ce résultat les tricots 3D décrits présentent des grammages et des épaisseurs élevées. Ceci conduit à la réalisation de bandes de contention plus volumineuses donc moins facile à manipuler car elles se présentent sous forme de bobineaux plus épais. Elles sont aussi plus lourdes ce qui augmentent le risque qu'elles glissent plus facilement au cours du temps.

Ainsi c'est pour palier ce défaut et l'absence de latex ou d'adhésif que le document WO 2009 / 71894 propose d'incorporer un adhésif ou un latex aux tricots 3D proposés dans le document WO 95 / 16416, ce qui pose donc également les problématiques précitées vis-à-vis de l'adhésif ou du latex en termes de mise au point et de risques d'allergie. Le document EP1052319 présente un autre exemple connu de tricot 3D.

La demande de brevet GB 2473321 propose la réalisation de tricots 3D toujours avec des grammages élevés pour s'approcher du rôle amortisseur de la ouate tout en appliquant des pressions et différentiels de pressions adaptés aux objectifs thérapeutiques. Toutefois tous les tricots décrits sont fabriqués selon la technologie « maille cueillie ». Du point de vue fabrication industrielle cette technologie n'est pas adaptée à la réalisation de bande de contention car la découpe d'une nappe de tricots 3D conduit au démaillage du produit. Le problème de glissement au cours du temps se pose également vis-à-vis de produits obtenus conformément à l'enseignement de ce document.

Pour y remédier, le document GB 2473321 propose de réaliser la « cohésivation » des tricots 3D par ajout de dérivés de silice seuls ou en association avec du latex ou des acrylates pour assurer le maintien au cours du temps, comme mentionné en page 14 de ce document, ce qui pose également les problématiques précitées en termes de mise au point et de risques d'allergie.

Le glissement d'une bande est causé par 3 facteurs primordiaux.

Le premier facteur est lié à la qualité de la pose. Si une bande est posée à un allongement trop faible elle risque de glisser car la pression appliquée sur le membre sera insuffisante pour la tenir en place. Un dispositif d'étalonnage permet de résoudre ce problème et d'éviter qu'à l'inverse si la bande est trop étirée on applique une pression trop élevée qui pourrait conduire à former un garrot. De même il est nécessaire de bien fixer la dernière spire pour éviter que la bande se détende à son extrémité puis sur la totalité de son enroulement ce qui va entrainer une perte de son efficacité thérapeutique voire son glissement le long de la jambe. Divers dispositifs sont utilisés pour renforcer cette fixation.

Le deuxième facteur est lié à la capacité de la bande à résister au glissement sur la peau qui est dépendant de son état de surface venant en contact avec cette dernière. Cet aspect est difficile à compenser car on souhaite avoir une face en contact avec la peau qui présente un toucher le moins désagréable possible pour favoriser l'observance du port de la bande par le patient.

Le troisième facteur est lié au mode de fonctionnement de la bande. Il consiste à trouver un équilibre entre la force appliquée par la bande en extension lors de la variation du diamètre du mollet et sa capacité à éviter le glissement latéral spire sur spire qui est illustré par leur relâchement intrinsèque et qui traduit le fait qu'elles se détendent par rapport à la pose. On retrouve alors le même phénomène que lors d'une pose déficiente c'est-à-dire une perte d'efficacité thérapeutique voire en cas de transmission de ce glissement latéral de spire en spire au cours du temps un glissement vertical de la bande qui peut à nouveau conduire à sa chute. Ce phénomène est amplifié par le poids de la bande.

Ce troisième facteur, cause de glissement, est particulièrement important et représente aussi la principale raison de la perte d'efficacité des systèmes de contention au cours du temps.

Paradoxalement cette cause de glissement n'a pas été étudiée de façon approfondie jusqu'à présent. Pour résoudre ce problème et s'opposer à ce relâchement intrinsèque, on a «cohésivé» les bandes c'est-à-dire privilégié l'incorporation d'adhésif ou du latex sur les bandes de contention. Ainsi dans tous les systèmes de contention à base de bandes à allongement court pour lesquelles ce phénomène est le plus important on incorpore aujourd'hui au moins une bande « cohésivée », posant à nouveau les problématiques définies précédemment.

En l'absence de cohésivation ou de tout autre moyen pour éviter ce phénomène de glissement si les 2 premiers facteurs sont maitrisés le troisième facteur devient alors essentiel .L'efficacité thérapeutique et le relâchement intrinsèque de la bande qui augmente la possibilité de glissement sont étroitement liés à cet équilibre et son évolution au cours du temps.

En conclusion bien que l'utilisation d'un tricot 3D comme bande de contention ai été proposée depuis bientôt 20 ans aucune solution n'apparait totalement satisfaisante pour obtenir un tricot 3D qui présente le comportement d'un allongement court pour permettre d'obtenir le bon différentiel de pression et, qui permette en l'absence de latex ou d'adhésif, de le conserver et d'éviter le risque de glissement le long du membre au cours du temps.

Pour résoudre ce cahier des charges très complexe avec des propriétés contradictoires le déposant a étudié les forces de frottement qui s'appliquent au niveau d'une bande en contact avec elle-même sous l'effet d'une pression correspondant à la pression de traitement thérapeutique recherchée par exemple dans le cas d'un ulcère de jambe de l'ordre de 35 à 50 mm de mercure. En effet le glissement des spires de la bande est lié aux micro-déplacements de cette dernière sur elle- même imposés par son poids à cause de la pesanteur et aux efforts de friction répétitifs induits par les variations du diamètre du mollet lors des mouvements.

Pour effectuer la mesure, qui n'avait jamais été envisagée, de ces micro-déplacements de la bande sur elle-même qui sont très faibles le déposant a utilisé un rhéomètre qui est un appareil qui classiquement sert à mesurer les propriétés rhéologiques de matériaux mous. Cet appareillage outre qu'il permet de déterminer des forces très faibles permet aussi d'appliquer un couple de cisaillement c'est-à-dire une torsion afin d'être représentatif des contraintes de frictions qui s'appliquent sur une bande à la fois dans les sens longitudinal et dans le sens transversal de cette dernière. La technique ainsi mise au point a permis de déterminer l'effort de cisaillement minimum responsable du premier micro-déplacement que subit la bande enroulée sur elle- même qui va conduire au glissement latéral des spires et au relâchement de la bande. Cet effort de cisaillement est appelé contrainte seuil de cisaillement car elle mesure le premier micro-déplacement et est exprimé en Pascal.

Cette mesure a permis de mieux comprendre les phénomènes mis en jeu et de déterminer les caractéristiques essentielles que doit présenter un tricot 3D pour remplir les propriétés énumérées ci-dessus et en particulier la contrainte seuil de cisaillement qu'il doit posséder pour éviter le relâchement intrinsèque de la bande pour conserver son efficacité thérapeutique et ne pas glisser.

Ainsi un tricot 3D, dans lequel le fil d'entretoise est un mono filament, qui présente une contrainte de seuil de cisaillement supérieure ou égale à 2800Pa permet de s'assurer du non glissement spire sur spire de la bande de contention.

Le déposant a notamment pu constater que dans un tricot 3D, le fil d'entretoise qui sépare et relie les deux surfaces textiles joue un rôle important.

Le déposant a en particulier pu constater que la rigidité du fil d'entretoise influe sur l'écart entre les deux surfaces textiles au repos et lors la déformation de la structure du tricot 3D quand ce dernier est soumis à des contraintes en extension lors de son utilisation en tant que bande de contention.

Or, une bande de contention doit à la fois être souple et confortable, la moins épaisse possible mais aussi présenter une certaine rigidité en extension.

Pour obtenir un produit qui puisse correspondre à ce cahier des charges complexe avec des propriétés antagonistes et optimiser le confort et la souplesse sans altérer les autres propriétés le déposant a étudié l'emploi comme fil d'entretoise de fils multi filaments.

Pour évaluer l'influence de tels fils multi filaments, le déposant a étudié leur rigidité. Pour cela le déposant a utilisé un modèle dans lequel le fil est considéré comme l'équivalent d'une poutre homogène à section circulaire.

Sur la base de ce modèle on a calculé le moment quadratique du fil qui va caractériser sa flexion et donc sa rigidité. Ce moment quadratique est exprimé en $m^4$ et défini comme :

$$I = n \times \pi \times D^4/64$$

Où n est le nombre de filaments du multi filaments (n=1 pour un mono filament), et D est le diamètre de chaque filament du multi filaments.

Le diamètre d'un fil (ou d'un filament) exprimé en micromètre ($\mu$m) est déterminable à partir du titre du fil exprimé en dtex (qui correspond à la masse linéique P de fil soit la masse de filen gramme (g) pour 10 000 mètres linéaires) soit $D = 20. \sqrt{\dfrac{P}{(\pi.d)}}$ Où d représente la densité du polymère utilisé dans le fil, par exemple 1,38 pour du polyester, 1,2 pour du polyamide et 0,9 pour du polypropylène, et P la masse linéique du fil ou du filament qui est exprimée en dtex.

Ainsi pour un mono filament de 50 dtex P =50 et pour un multi filaments de 50/24 dtex le poids du filament est 50/24 soit 2,08.

En utilisant ce modèle on a ainsi calculé pour un mono filament et un multi filament ayant le même titre en dtex le moment quadratique et pour les multi filaments en fonction du dtex du fil et du nombre de filaments par exemple 50/24 dtex les moments quadratiques par exemple pour un fil en polyester.

Les résultats sont rassemblés ci-après, et confirment que le moment quadratique d'un mono filament est supérieur à celui d'un multi filaments.

Ainsi pour les fils suivants on trouve les résultats ci -dessous.

Mono filament 22 dtex I = 2 10$^{-19}$

Multi filaments 22/12 dtex I = 1,7 10$^{-20}$

Mono filament 50dtex I = 1,04 10$^{-18}$

Multi filaments 50/24 dtex I = 4,35 10$^{-20}$

Mono filament 55 dtex I = 1,26 10$^{-18}$

Multi filaments 55/48 dtex I = 2,60 10$^{-20}$

Monofilament 80 dtex I= 2,7 10$^{-18}$

Multi filaments 80/70 dtex I = 3,82 10$^{-20}$.

On constate donc des variations importantes de ce moment quadratique entre 2 fils de même titre. Ainsi, le moment quadratique d'un mono filament est de l'ordre de 12 fois supérieur à celui d'un multi filaments pour le 22dtex, 24 fois pour le 50dtex, 48 fois pour le 55dtex et 70 fois pour le 80 dtex.

Il ressort donc de cette analyse que le comportement et les caractéristiques d'un tricot 3D à base de multi filaments ne seront pas similaires à ceux d'un tricot 3D avec mono filament.

Pour favoriser la souplesse et le confort l'usage d'un multi filament semble préférable.

Afin d'obtenir le produit le plus souple et le plus confortable possible malgré une épaisseur faible pour faciliter la manipulation de la bande le déposant a donc étudié le comportement d'un tricot 3D avec comme fil d'entretoise un multi filaments et déterminer quelles caractéristiques devait posséder un tel tricot 3D pour pouvoir être utilisé sans ajout d'adhésif ou de latex comme bande de contention et son influence sur le glissement spire sur spire.

Comme indiqué précédemment, la principale différence entre de tels fils multi filaments et des mono filament est que les fils multi filaments présentent une rigidité inférieure à celle d'un mono filament à titre équivalent.

Leur comportement et leur influence dans une structure textile n'est donc ni similaire ni prévisible par rapport à un tricot qui possède comme fil d'entretoise un mono filament. Ceci est d'autant plus important dans un tricot 3D que le multi filaments sert de fil d'entretoise qui à la fois sépare et relie les 2 faces textiles.

Les résultats des tests décrits ci - après ont confirmé cette différence de comportement. Ils ont démontré, qu'avec un fil d'entretoise qui est un multi filament, la contrainte de seuil de cisaillement n'est pas nécessairement l'unique paramètre qui entre en jeu. Ainsi des tricots 3D, pour lesquels la contrainte de seuil de cisaillement est inférieure à 2800Pa, permettent aussi de s'assurer du non glissement spire sur spire de la bande de contention.

Bien que les phénomènes physiques mis en jeu soient très complexes le déposant a constaté que la capacité du tricot 3D, par rapport à un tricot dans lequel le fil d'entretoise est un mono filament, a parfaitement épouser la forme du membre est aussi un paramètre essentiel qui semble soit s'ajouter à l'effet de la contrainte de cisaillement soit la compenser si cette dernière est trop faible.

Ce contact intime atténuerait les contraintes de friction qui s'appliquent à la bande, à la fois dans le sens longitudinal et dans le sens transversal, lors du mouvement du mollet, et permettrait ainsi de diminuer les micros déplacements de la bande sur elle-même et d'éviter le glissement spire sur spire.

Cette adaptabilité d'un tricot 3D n'a jamais été étudiée ni mesurée.

La souplesse d'un matériau textile ne peut être évaluée qu'à partir de tests uniaxiaux car toute modification de ses propriétés dans une direction interfère dans les autres directions. Ce phénomène est d'autant plus complexe avec un tricot 3D que ce produit peut varier suivant les 3 axes x, y et z.

Pour déterminer la caractéristique technique qui permet d'obtenir cette propriété d'adaptation à la forme et au mouvement du membre le déposant a adapté une technologie utilisée dans la pharmacopée britannique qui permet de mesurer la déformation d'un pansement hydrocolloïde imperméable aux liquides. Le principe consiste à mesurer la déformation d'un pansement imperméable sous une pression induite par de l'air comprimé.

On mesure ainsi le rayon de courbure du pansement quand il est soumis à une pression donnée.

Le déposant a adapté ce test pour les tricots 3D qui ne sont pas imperméables à l'air. Ce test bi axial de déformation s'est avéré être très adapté pour mesurer la capacité du tricot 3D à s'adapter à la forme et au mouvement du corps. C'est la mesure de ce rayon de courbure du tricot 3D que l'on a qualifiée de conformabilité du tricot 3D.

Grâce à la mise au point de ce nouveau test il a pu déterminer toutes les caractéristiques techniques en termes de contrainte de seuil de cisaillement et de conformabilité que doit posséder un tricot 3D, dans lequel le fil d'entretoise est un multi filament, et qui permettent de s'assurer du non glissement spire sur spire pour ce type de bande de contention.

La présente invention est donc relative à une bande dont l'allongement longitudinal est compris entre 30% et 160%, qui est un tricot 3D dont le fil d'entretoise est un multi filaments, obtenu selon la technologie « maille jetée », sans latex ni adhésif, qui ne glissent pas pendant au moins 48 heures et mieux au moins pendant 3 jours ou plus. En effet dans le cadre du traitement des ulcères de jambes qui présentent des plaies très exsudatives ces durées minima de 48 et 72 heures correspondent aux durées habituelles de changement des pansements qui sont disposés sous les bandes de contention. Il est donc indispensable que la bande reste en place au moins 2 ou 3 jours sans glisser.

PRESENTATION DE L'INVENTION

La présente invention est donc relative à une bande de contention qui se présente sous la forme d'un tricot obtenu par la technologie maille jetée, à base de fils synthétiques qui est constitué de 2 surfaces textiles dont la structure textile est identique ou différente reliées entre elles par des fils d'entretoises qui sont des multi filaments, chaque surface textile comportant des fils élastiques, caractérisé en ce que ledit tricot présente un allongement longitudinal mesuré selon la norme EN 14704 -1 compris entre 30 et 160% et une contrainte de seuil de cisaillement supérieure ou égale à 2800 Pa, et/ou une conformabilité inférieure ou égale à 65 mm.

Des tests décrits ci - après ont démontré qu'une bande possédant au moins une contrainte de seuil de cisaillement ou une conformabilité telle que mentionnée précédemment permet de s'assurer du non glissement spire sur spire de la bande de contention et ainsi d'éviter son relâchement intrinsèque et donc de conserver son efficacité thérapeutique et d'empêcher son glissement.

Selon la présente invention le tricot 3D pourra être à usage unique ou réutilisable et donc par conséquent lavable.

Après l'opération de tricotage, afin de stabiliser le tricot 3D, notamment afin d'obtenir un produit lavable, sa structure sera figée en utilisant les technologies couramment employées à cet effet telle la thermo fixation par la chaleur ou un traitement de vaporisage. Ces opérations consistent à faire passer dans une étape supplémentaire, en ligne avec le tricotage ou séparée de cette dernière, à une vitesse donnée et une température fixée le tricot dans un four pour la thermo fixation et à travers un flux de vapeur d'eau pour le vaporisage.

Afin de favoriser une pose précise par le personnel soignant, la bande de contention peut être munie d'un moyen d'étalonnage. Ce moyen d'étalonnage peut être visuel, comme par exemple un ensemble de pictogrammes, régulièrement espacés, imprimés sur la bande ou réalisés au moyen d'un système d'étalonnage. Des informations sur les allongements à la pose recommandés peuvent être fournies avec le moyen d'étalonnage. L'étalonnage peut aussi être réalisé par le personnel soignant sous la forme d'un pochoir. Ce type de pochoir ou les explications nécessaires pour le fabriquer peuvent être incorporés dans le conditionnement de la bande. Un kit comprenant plusieurs bandes de constitutions différentes, de largeurs différentes, de longueurs différentes et/ou dotées d'étalonnages différents pour appliquer des pressions spécifiques pourra être utilisé.

Le kit pourra en outre comprendre un ou plusieurs pansements destinés à être posés sur la plaie avant la pose de la bande.

Pour favoriser la facilité de manipulation lors de la pose on choisira un tricot qui présente un allongement longitudinal tel que défini dans la norme EN 14704-1 qui soit compris entre 40 et 160%, ou plus précisément entre 50 et 120 %, ou encore plus précisément entre 55 et 100%.

Le tricot présente par exemple une épaisseur comprise entre 1 et 2 mm, ou plus précisément entre 1 et 1,5 mm.

Le tricot présente par exemple un grammage compris de 160 à 370 g/m², ou plus précisément de 180 à 300 g/m², ou encore plus précisément de 200 à 250 g/m².

De même de préférence le tricot présente par exemple un écart entre les 2 faces textiles compris entre 0,4 et 1,5mm, ou plus précisément entre 0,5 et 1,1mm.

Ces propriétés de faible grammage et d'épaisseur assurent une utilisation facilitée avec les chaussures de la bande de contention. La bande de contention pourra ainsi être aussi plus facilement utilisée avec une ouate si nécessaire.

Les deux surfaces textiles du tricot peuvent avoir des structures textiles identiques ou différentes. Ces structures textiles peuvent être pleines ou ajourées.

Les structures textiles ajourées appelées tricot à jours et désignées dans la présente demande sous le terme de filet sont bien connues par l'homme du métier. Un tricot à jours est un tricot qui présente des trous réguliers ou irréguliers dans sa structure textile. Ces trous sont obtenus lorsque, dans la structure textile, une ou plusieurs mailles d'une colonne ne sont pas reliées aux mailles de la colonne voisine lors du tricotage, typiquement en jouant sur le schéma de maille et / ou sur l'enfilage.

Selon un aspect de la présente invention le tricot présente deux surfaces textiles dont la structure textile est différente et en particulier une surface textile qui présente une structure textile ajourée appelée face filet et une surface textile qui présente une structure textile face pleine. La présence d'une face filet permet de favoriser la respirabilité de la bande. Une telle face filet est typiquement disposée au contact de la peau d'un utilisateur.

Selon un mode de réalisation particulier, ledit tricot présente une face qui a une structure textile de type charmeuse, drap, demi-simple à mailles ouvertes ou fermées, atlas sous un ou plusieurs rangs, ou chainette à mailles ouvertes, fermées, ou à alternance de mailles fermées et ouvertes. Cette face est opposée à la face adaptée pour être mise en contact avec la peau, qui présente une structure textile qui est un filet avec le même type ou un type différent de structure textile ajourée.

Afin de faciliter le passage du talon et éviter une striction de la bande lors de la pose on peut utiliser des tricots 3D qui présentent un allongement transversal supérieur à 120 % tel que mesuré selon la méthode A §9.1 de la norme EN 14704-1, ou par exemple compris entre 120% et 300%, ou encore entre 120% et 250%.

Les tricots selon l'invention sont par exemple réalisés à l'aide de fils couramment employés dans la réalisation des produits textiles et en particulier des tricots. Ces fils sont par exemple synthétiques. Ces fils se partagent en 2 grandes catégories les fils élastiques et les fils thermoplastiques.

Parmi les fils élastiques on peut par exemple citer les fils à base de fibres polyuréthanne comme les fils d'Elasthanne commercialisé sous la dénomination LYCRA, les fils à base d'élastodiène ou les fils à base de polymères triblocs (styrène - éthylène - butylène - styrène).

Parmi les fils thermoplastiques on peut citer les fils constitués à base de matériaux synthétiques qui ne sont pas des élastomères comme par exemple le polyester, le polyamide, le polypropylène, le polybutylène terephtalate (PBT).

Tous ces fils thermoplastiques peuvent être guipés ou non, texturés ou non.

Les deux surfaces textiles du tricot 3D sont par exemple réalisées à partir de fils élastiques et de fils thermoplastiques. Ces fils peuvent être mono filament ou multi filaments. Ces surfaces textiles pourront être réalisées à partir de fils identiques ou différents. Les deux surfaces comprendront de préférence des fils élastiques similaires.

Les fils élastiques présents sur ces surfaces textiles présentent par exemple des titres de l'ordre de 40 à 80 dtex et les fils thermoplastiques des titres de 40 à 90 dtex.

Si l'on souhaite favoriser le transfert d'humidité du tricot vers l'extérieur on pourra utiliser des fils de nature non synthétique, comme par exemple le coton ou la viscose, sur une des deux faces en particulier celle en contact avec la peau.

On utilise par exemple comme fil élastique des fils d'élasthanne et comme fil thermoplastique des fils de polyamide ou de polyester.

Les fils d'entretoises sont typiquement des fils thermoplastiques multi filaments, comme par exemple des fils en polyester, en polypropylène ou en polyamide.

On entend par multi filament un fil constitué de l'association de plusieurs filaments continus liés entre eux par exemple par torsion, entremêlement, guipage ou points de collage. Ces fils sont définis par le titre du fil exprimé en dtex et le nombre de filaments qui le composent par exemple 50/24 dtex ce qui signifie un fil de 50dtex constitué de 24 filaments. De façon générale ces fils sont plus fragiles que les fils mono filament et ceci d'autant plus que les filaments auront un faible diamètre. Ainsi pour les fils présentant un faible dtex ils sont plus difficiles à tricoter. A l'inverse plus le nombre de filaments sera important par rapport à un mono filament pour un même dtex plus le fil sera épais et donc à nouveau difficile à tricoter.

Dans le cadre de la présente invention on choisira donc, de préférence, des fils multi filaments compris entre 20 et 85 dtex et dans lesquels le filament n'est pas une microfibre c'est-à-dire que son titre est supérieur ou égal à 1 dtex. On préférera tout particulièrement un multi filaments qui présente un titre compris entre 20 et 80 dtex, ou encore entre 33 et 80 dtex, ou encore entre 40 et 80 dtex, ou encore entre 40 et 70 dtex, et un nombre de filaments supérieur à 12 ou encore plus précisément un multi filaments en polyester qui présente un titre entre 44 et 55 dtex et un nombre de filament entre 12 et 24.

Pour la réalisation du tricot 3D, on peut par exemple utiliser une seule barre pour tricoter le fil d'entretoise qui lie les 2 surfaces textiles.

L'invention concerne également un kit comprenant une ou plusieurs bandes de contention telles que définies précédemment, et un ou plusieurs pansements adaptés pour être disposés sur une plaie préalablement à l'une des bandes de contention.

## DESCRIPTION DETAILLEE

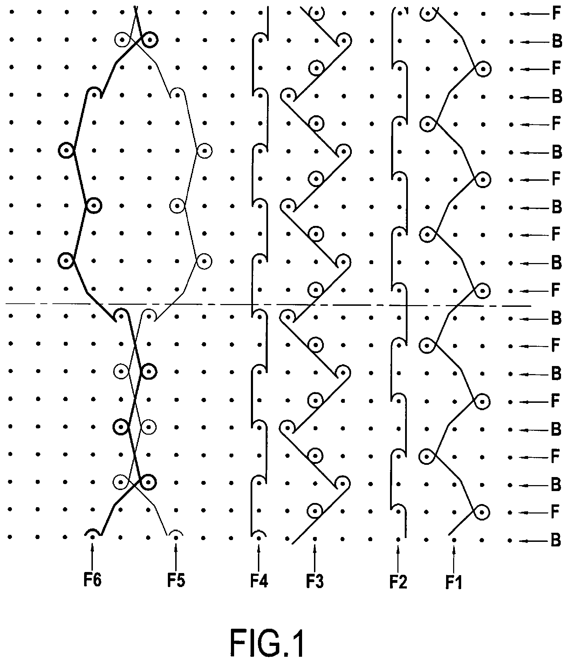

L'invention sera illustrée par les exemples et les tests comparatifs suivants, ainsi que par les figures 1 à 5.

### Exemple de mise en œuvre de l'invention

On a fabriqué un tricot d'environ 10 cm de largeur selon l'invention sur un métier Raschel maille jetée double fonture jauge 22.

Ce tricot présente une face qui vient en contact avec la peau qui est un filet et la face opposée une face pleine.

Pour réaliser le tricot on a utilisé 6 barres selon le schéma de maille représenté sur la figure 1 avec les fils et les conditions suivantes :

### Nature des fils

- F1 : fil de polyamide commercialisé par la société RADICI sous la référence 78/18/1 dtex S Beige
- F2 : fil d élasthanne de 44 dtex commercialisé par la société ASAHI KASEI GROUP
- F3 : fil qui est un multi filament de polyester de 50/24 dtex commercialisé par la société SINTERAMA
- F4 : fil d'élasthanne de 44 dtex commercialisé par la société ASAHI KASEI GROUP
- F5 : fil de polyamide 66 commercialisé par la société EMILE TARDY sous la référence PA 66 1/ 44/34/ FT BE MM
- F6 : fil de polyamide 66 commercialisé par la société EMILE TARDY sous la référence PA 66 1/ 44/34/ FT BE MM

### Réglages du métier à tricoter

- F1 : alimentation 2400 mm de fils consommés pour la réalisation de 480 mailles - enfilage plein
- F2 : alimentation 1300mm de fils consommés pour la réalisation de 480 mailles - enfilage 1plein/1 vide
- F3 : alimentation 3600 mm de fils consommés pour la réalisation de 480 mailles - enfilage plein
- F4 : alimentation 2000mm de fils consommés pour la réalisation de 480 mailles - enfilage 1plein/1 vide
- F5: alimentation 2100 mm de fils consommés pour la réalisation de 480 mailles- enfilage 3pleins/1vide
- F6 : alimentation 2100 mm de fils consommés pour la réalisation de 480 mailes -enfilage 3pleins/1vide

### Schéma de maille

On représente sur la figure 1 le graphique d'un exemple de structure de maille pour la réalisation d'un tricot selon un mode de réalisation particulier de l'invention.

Sur cette figure, on représente la fonture avant par la référence F, et la fonture arrière par la référence B. Les schémas de maille des fils F1 à F6 sont ensuite illustrés.

On comprend bien que cet exemple ainsi que les exemples suivant sont purement illustratifs, et qu'ils ne doivent pas être interprétés de manière limitative quant à la portée de l'invention.

Le tricot ainsi réalisé subit une étape de traitement par la chaleur en ligne.

Durant cette étape le tricot passe en une seule fois entre 2 rouleaux formés de cylindres chauffants de manière à ce que chaque face subisse ce traitement à la vitesse de 5,5 mètres par minute. La température des cylindres avant le passage du tricot est réglée pour être de l'ordre de 165 °C.

Les techniques suivantes ont été utilisées pour évaluer les paramètres du tricot obtenu.

### Mesure du grammage

La mesure du grammage est réalisée selon la norme NF EN 12127. On pèse 5 éprouvettes d'une surface de 100 cm$^2$ (mesure = +/- 1 %) avec une balance dont la précision est au plus de 1 mg.
La pesée est réalisée à une température de 21 °C +/-2 °C et 60 % +/- 15 % HR.
La mesure finale est une moyenne des 5 éprouvettes.

### Mesure de l'épaisseur

La mesure de l'épaisseur s'effectue selon la norme NF EN ISO 9073-2. On utilise un micromètre laser KEYENCE, (muni d'une tête de capteur laser CCD LK-G87 et d'un capteur de déplacement laser CCD LK-G3001PV). La pression d'application est fixée à 0,5kPa et la surface du disque en acier est de 2500mm$^2$.

### Mesure de l'écart entre les faces

Cette mesure est réalisée de la façon suivant.
A l'aide d'un microscope numérique KEYENCE (optiques x 100 ou x 200) on détermine l'espace entre les deux plans des 2 surfaces textiles.
Le plan moyen des 2 surfaces est matérialisé par un trait horizontal estimé par l'opérateur et la distance entre les deux traits est déterminée de manière automatique par le logiciel .La mesure est reproduite plusieurs fois afin d'augmenter la précision et on réalise une moyenne des mesures obtenues.

### Mesure de la contrainte seuil de cisaillement

Les mesures sont réalisées à l'aide d'un rhéomètre DHR2 commercialisé par la société TA Instruments.
Elles sont effectuées à une température de 35°C (de manière à être proche de la température des bandes au contact de la peau) ladite température étant régulée par un plan Peltier qui équipe le rhéomètre. On découpe 2 disques de 25 mm de diamètre du tricot 3D analysé.
Ces 2 disques sont collés respectivement à l'aide d'un adhésif double face fin et rigide commercialisé par la société Plasto sous la référence P753 sur la face métallique du plateau mobile et du plateau du plan Peltier du rhéomètre. On met en contact les 2 disques de tricot 3D, face structure charmeuse (également appelée structure drap) sur face structure filet, en appliquant une pression de 5,3 kPa (soit l'équivalent de 40mm de mercure). Le programme de pilotage du rhéomètre génère une rampe de contrainte (couple de torsion) qui varie de 100 à 10 000 Pa en 600 secondes. L'appareil enregistre le premier micro-déplacement qu'il détecte qui correspond à la contrainte seuil de cisaillement exprimée en Pa.
On considère que l'incertitude instrumentale sur cette mesure est plus ou moins 6%.
La mesure finale est la moyenne des valeurs obtenues pour 5 échantillons du même tricot 3D.

### Mesure de la conformabilité

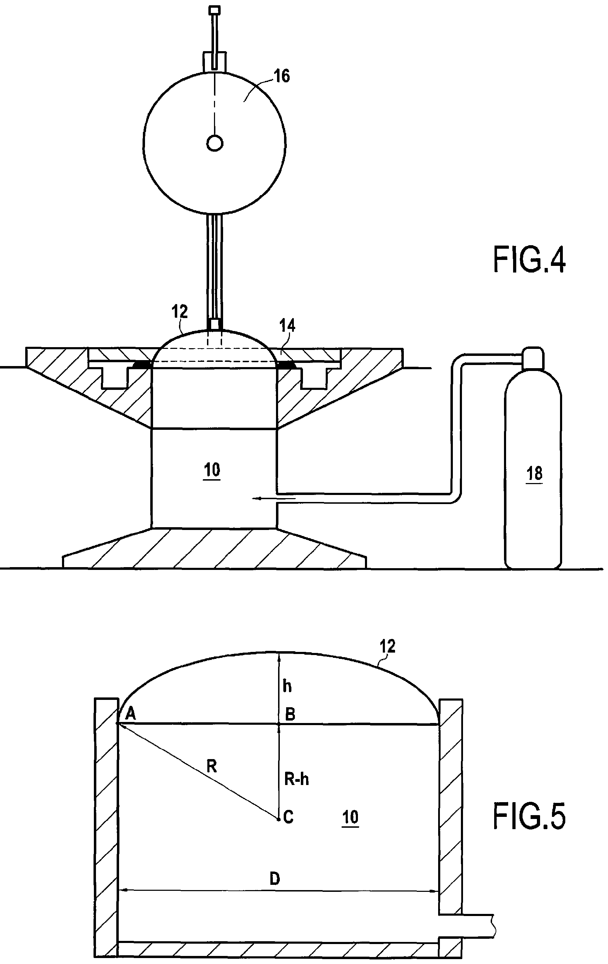

La description de ce test est illustrée par les figures 4 et 5.
Comme indiqué sur la figure 4 on utilise une cellule de mesure de la déformation qui est un cylindre de 75 mm de diamètre alimenté en air comprimé à une pression de 50 mbars.
On laisse les tricots 3D à tester en enceinte conditionnée pendant au moins 24 heures à 21 plus ou moins 2°C et 60 plus ou moins 15% d'humidité relative. On découpe un échantillon 12 de tricot 3D à l'aide d'un emporte- pièce de 99 mm de diamètre. Le tricot 3D étant perméable à l'air on découpe un film de polyuréthane très fin de 30 micromètres du même diamètre. Ce dernier permet d'obtenir le caractère imperméable à l'air pour faire la mesure. Il possède un niveau de déformation très important par rapport au tricot 3D et sa présence ne modifie pas les résultats obtenus De plus tous les tricots sont testés en présence de ce dernier de façon comparative.
On pose le film de polyuréthane recouvert du tricot 3D sur la cellule de mesure (10) et on pince dans un plan de joint par un dispositif de pression 14 de manière à rendre le système étanche à l'air.
On ajuste avec un micromètre 16 à 0 mm la surface de l'échantillon 12. On règle le débit d'air (fourni par une source d'alimentation en air comprimé 18) et on laisse l'échantillon 12se stabiliser pendant au moins une minute. Le tricot se déforme et forme une calotte sphérique dont on mesure la hauteur h à l'aide du micromètre 16.
Cette déformation est exprimée par le calcul du rayon de courbure formé par le tricot 3D sous la pression de l'air comprimé.
Son calcul est réalisé sur la base de cette hauteur h et du diamètre D de la cellule de mesure comme illustré sur la figure 5.

$$R= h/2 + D^2/8h.$$

Ce rayon de courbure R (que l'on qualifie de conformabilité) est exprimé en mm.
On reproduit cette mesure sur 7 échantillons du même tricot 3d et la valeur finale est la moyenne de ces 7 mesures.
Les paramètres du tricot obtenu sont les suivantes (Exemple 1) :

- Grammage : 234g/m$^2$
- Epaisseur : 1,39 mm
- Contrainte seuil de cisaillement : 2227 Pa
- Conformabilité : 63,6 mm
- Ecart entre les faces : 0,9 mm
- Allongement longitudinal selon la norme EN 14704 - 1 : 73 %
- Allongement transversal selon la norme EN 14704 - 1 : 144 %

On a également réalisé plusieurs autres exemples de tricot, que l'on détaille ci-après.
Ces autres exemples sont réalisés au moyen d'un schéma de maille identique à celui détaillé pour l'Exemple 1 (sauf indication contraire). On détaille ci-après la nature des fils et le réglage du métier à tricoter, ainsi que les caractéristiques obtenues.
**Exemple 2 :** correspondant par exemple à un produit qui comme l'exemple 1 présente une face qui vient en contact avec la peau qui est un filet et la face opposée une face pleine et qui est un allongement long :
Une bande de produit obtenue dans l'exemple 1 a ensuite été soumise à 5 lavages successifs, sans séchage entre les lavages, dans une machine à laver à 40°C et 800 tours/minutes, avec une lessive commercialisée sous la marque commerciale « Le Chat machine ».

**Caractéristiques du produit obtenu :(Exemple 2)**

- Grammage : 315/m$^2$
- Epaisseur : 1,6 mm
- Contrainte seuil de cisaillement : 3667 Pa
- Conformabilité : 70,3 mm
- Ecart entre les faces : 1,17 mm
- Allongement longitudinal selon la norme EN 14704 - 1 : 112 %
- Allongement transversal selon la norme EN 14704 - 1 : 125 %

**Exemple 3** : correspondant par exemple à un produit sans face ajourée c'est-à-dire avec 2 faces pleines :

**Nature des fils** :

- F1 : fil de polyamide commercialisé par la société RADICI sous la référence 78/18/1 dtex S Beige
- F2 : fil d'élasthanne de 44 dtex commercialisé par la société ASAHI KASEI GROUP
- F3 : fil qui est un multi filaments de polyester de 50/24 dtex commercialisé par la société SINTERAMAFILVA
- F4 : fil d'élasthanne de 44 dtex commercialisé par la société ASAHI KASEI GROUP
- F5 : fil de polyamide 66 commercialisé par la société Defiber sous la référence PA 66 1/ 44/34/ dTex
- F6 : fil de polyamide 66 commercialisé par la société Defiber sous la référence PA 66 1/ 44/34/ dTex

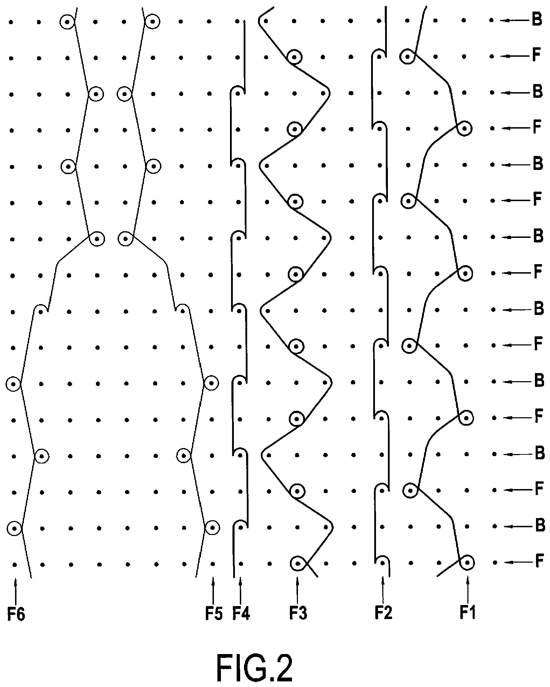

Le schéma de maille est ici distinct de celui des autres exemples, et est représenté sur la figure 2.

**Réglage du métier à tricoter** :

- F1 : alimentation 2700 mm de fils consommés pour la réalisation de 480 mailles - enfilage plein
- F2 : alimentation 1300mm de fils consommés pour la réalisation de 480 mailles - enfilage 1plein/1 vide
- F3 : alimentation 3600 mm de fils consommés pour la réalisation de 480 mailles - enfilage plein
- F4 : alimentation 1700mm de fils consommés pour la réalisation de 480 mailles - enfilage 1plein/1 vide
- F5: alimentation 2000 mm de fils consommés pour la réalisation de 480 mailles- enfilage 3pleins/1vide

- F6 : alimentation 2000 mm de fils consommés pour la réalisation de 480 mailes -enfilage 3pleins/1vide

Le tricot ainsi réalisé subit ensuite une étape de traitement par la chaleur en ligne.

Durant cette étape le tricot passe en une seule fois entre 2 rouleaux formés de cylindres chauffants de manière à ce que chaque face subisse ce traitement à la vitesse de 5 mètres par minute. La température des cylindres avant le passage du tricot est réglée pour être de l'ordre de 165 °C.

**Caractéristiques du produit obtenu :(Exemple 3)**

- Grammage : 264/m$^2$
- Epaisseur : 1,5 mm
- Contrainte seuil de cisaillement : 2217 Pa
- Conformabilité : 63,3 mm
- Ecart entre les faces : 1,21 mm
- Allongement longitudinal selon la norme EN 14704 - 1 : 89 %
- Allongement transversal selon la norme EN 14704 - 1 : 176 %

**Exemple 4** : correspondant par exemple à un produit sans face ajourée c'est-à-dire avec 2 faces pleines :

**Nature des fils** :

- F1 : fil de polyamide commercialisé par la société RADICI sous la référence 78/18/1 dtex S Beige
- F2 : fil d'élasthanne de 44 dtex commercialisé par la société ASAHI KASEI GROUP
- F3 : fil qui est un multi filament de polyester de 50/24 dtex commercialisé par la société SINTERAMAFILVA
- F4 : fil d'élasthanne de 44 dtex commercialisé par la société ASAHI KASEI GROUP
- F5 : fil de polyamide 66 commercialisé par la société Defiber sous la référence PA 66 1/ 44/34/ dTex
- F6 : fil de polyamide 66 commercialisé par la société Defiber sous la référence PA 66 1/ 44/34/ dTex

Le schéma de maille est ici identique à celui de l'exemple 3, et est représenté sur la figure 2.

**Réglage du métier à tricoter** :

- F1 : alimentation 2700 mm de fils consommés pour la réalisation de 480 mailles - enfilage plein
- F2 : alimentation 1300mm de fils consommés pour la réalisation de 480 mailles - enfilage 1plein/1 vide
- F3 : alimentation 3600 mm de fils consommés pour la réalisation de 480 mailles - enfilage plein
- F4 : alimentation 1700mm de fils consommés pour la réalisation de 480 mailles - enfilage 1plein/1 vide
- F5: alimentation 2000 mm de fils consommés pour la réalisation de 480 mailles- enfilage 3pleins/1vide
- F6 : alimentation 2000 mm de fils consommés pour la réalisation de 480 mailes -enfilage 3pleins/1vide

Le tricot ainsi réalisé subit ensuite une étape de traitement par la chaleur en ligne.

Durant cette étape le tricot passe en une seule fois entre 2 rouleaux formés de cylindres chauffants de manière à ce que chaque face subisse ce traitement à la vitesse de 3,75 mètres par minute. La température des cylindres avant le passage du tricot est réglée pour être de l'ordre de 165 °C.

**Caractéristiques du produit obtenu :(Exemple 4)**

- Grammage : 267/m$^2$
- Epaisseur : 1,6 mm
- Contrainte seuil de cisaillement : 2207 Pa
- Conformabilité : 67,1 mm

- Ecart entre les faces : 1,46 mm
- Allongement longitudinal selon la norme EN 14704 - 1 : 93 %
- Allongement transversal selon la norme EN 14704 - 1 : 174 %

**Exemple 5** : correspondant par exemple à un produit ayant une face au contact de la peau qui est un filet et la face opposée une face pleine :

**Nature des fils** :

- F1 : fil de polyamide commercialisé par la société RADICI sous la référence 78/18/1 dtex S Beige
- F2 : fil d'élasthanne de 44 dtex commercialisé par la société ASAHI KASEI GROUP
- F3 : fil qui est un multifilament de polyester de 50/24 dtex commercialisé par la société SINTERAMAFILVA
- F4 : fil d'élasthanne de 44 dtex commercialisé par la société ASAHI KASEI GROUP
- F5 : fil de polyamide 66 commercialisé par la société Defiber sous la référence PA 66 1/ 44/34/ dTex
- F6 : fil de polyamide 66 commercialisé par la société Defiber sous la référence PA 66 1/ 44/34/ dTex

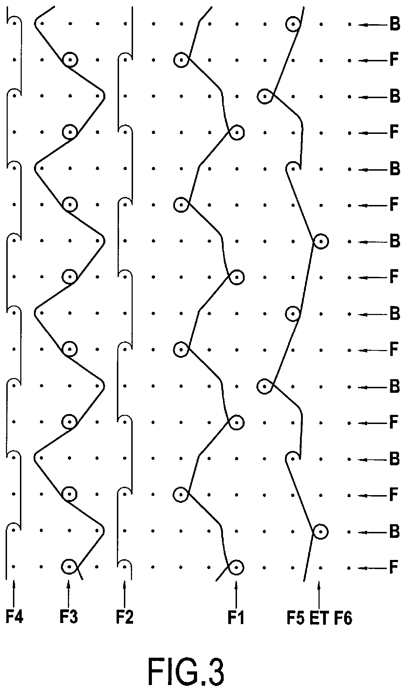

Le schéma de maille est ici distinct des autres exemples et est représenté sur la figure 3.

**Réglage du métier à tricoter** :

- F1 : alimentation 2700 mm de fils consommés pour la réalisation de 480 mailles - enfilage plein
- F2 : alimentation 1300mm de fils consommés pour la réalisation de 480 mailles - enfilage 1plein/1 vide
- F3 : alimentation 4000 mm de fils consommés pour la réalisation de 480 mailles - enfilage plein
- F4 : alimentation 1700mm de fils consommés pour la réalisation de 480 mailles - enfilage 1plein/1 vide
- F5: alimentation 2000 mm de fils consommés pour la réalisation de 480 mailles- enfilage 3pleins/1vide
- F6 : alimentation 2000 mm de fils consommés pour la réalisation de 480 mailes -enfilage 3pleins/1vide

Le tricot ainsi réalisé subit ensuite une étape de traitement par la chaleur en ligne.
Durant cette étape le tricot passe en une seule fois entre 2 rouleaux formés de cylindres chauffants de manière à ce que chaque face subisse ce traitement à la vitesse de 5,5 mètres par minute. La température des cylindres avant le passage du tricot est réglée pour être de l'ordre de 165 °C.

**Caractéristiques du produit obtenu :(Exemple 5)**

- Grammage : 274/m$^2$
- Epaisseur : 1,7 mm
- Contrainte seuil de cisaillement : 1707 Pa
- Conformabilité : 67,4 mm
- Ecart entre les faces : 1,32 mm
- Allongement longitudinal selon la norme EN 14704 - 1 : 88 %
- Allongement transversal selon la norme EN 14704 - 1 : 168 %

**Exemple 6** : correspondant à un tricot avec un monofilament

On a fabriqué un tricot d'environ 10 cm de largeur selon l'invention sur un métier Raschel maille jetée double fonture jauge 22.
Ce tricot présente une face qui vient en contact avec la peau qui est un filet et la face opposée une face pleine.

**Nature des fils**

- **F1** : fil de polyamide commercialisé par la société RADICI sous la référence 78/18/1 dtex S Beige
- **F2** : fil d élasthanne de 44 dtex commercialisé par la société ASAHI KASEI GROUP
- **F3** : fil qui est un monofilament de polyester de 55 dtex commercialisé par la société FILVA
- **F4** : fil d'élasthanne de 44 dtex commercialisé par la société ASAHI KASEI GROUP
- **F5** : fil de polyamide 66 commercialisé par la société EMILE TARDY sous la référence PA 66 1/ 44/34/ FT BE MM
- **F6** : fil de polyamide 66 commercialisé par la société EMILE TARDY sous la référence PA 66 1/ 44/34/ FT BE MM

**Réglages du métier à tricoter**

- **F1** : alimentation 2500 mm de fils consommés pour la réalisation de 480 mailles - enfilage plein
- **F2** : alimentation 1500mm de fils consommés pour la réalisation de 480 mailles - enfilage 1plein/1 vide
- **F3** : alimentation 3500 mm de fils consommés pour la réalisation de 480 mailles - enfilage plein
- **F4** : alimentation 1600mm de fils consommés pour la réalisation de 480 mailles - enfilage 1plein/1 vide
- **F5**: alimentation 2250 mm de fils consommés pour la réalisation de 480 mailles- enfilage 3pleins/1vide
- **F6** : alimentation 2250 mm de fils consommés pour la réalisation de 480 mailes -enfilage 3pleins/1vide

**Schéma de maille**

Le schéma de maille est ici identique à celui de l'exemple 1 et est donc représenté sur la figure 1.
Le tricot ainsi réalisé subit une étape de traitement par la chaleur en ligne.
Durant cette étape le tricot passe en une seule fois entre 2 rouleaux formés de cylindres chauffants de manière à ce que chaque face subisse ce traitement à la vitesse de 5,5 mètres par minute. La température des cylindres avant le passage du tricot est réglée pour être de l'ordre de 190 °C.

**Caractéristiques du produit obtenu :(Exemple 6)**

- Grammage : 232g/m$^2$
- Epaisseur : 1,23 mm
- Contrainte seuil de cisaillement : 3080 Pa
- Conformabilité : 66,2 mm
- Ecart entre les faces : 0,64 mm
- Allongement longitudinal selon la norme EN 14704 - 1 : 56 %
- Allongement transversal selon la norme EN 14704 - 1 : 128 %

On a ensuite comparé les performances de pression in vitro, selon le test décrit ci-après, entre les exemples 3 et 6 selon l'invention et le système de contention bicouche commercialisés sous la dénomination K2 par la société Laboratoires URGO.

**Test in vitro**

Les performances du tricot 3D des exemples 3 et 6 et du système de contention bicouche commercialisés sous la dénomination K2 par la société Laboratoires URGO ont été évaluées en termes de pressions de travail et de repos et de différentiel de pression, au cours du temps.
On a utilisé la méthode et l'appareil de test in vitro décrit dans la demande de brevet WO 2007/113430 page 17 ligne 26 à page 19 ligne 18. Selon cette méthode la bande est posée autour d'un cylindre avec un recouvrement total de 100%, puis on fait varier la circonférence du cylindre à une vitesse imposée de façon continue entre une position dite de repos (diamètre le plus faible) et une position dite de travail (diamètre le plus grand) pour mimer la contraction musculaire. Des capteurs de pression mesurent au cours du temps les valeurs des pressions de repos et des pressions de travail.
L'écart de temps entre les mesures de pression de travail et de pression de repos est de 5 secondes et la fréquence des mesures de ces deux paramètres successifs est de 0,2Hz.
Pour tester les bandes de contention selon l'invention on a déterminé l'allongement à la pose de la bande en fonction de la pression de travail recherchée, par exemple à l'aide de la courbe traction rupture telle que définie dans la norme EN ISO 13934-1. D'après la loi de Laplace, l'allongement à effectuer correspond à la pression recherchée.

On découpe une bande rectangulaire de largeur suffisante en l'effilochant si besoin pour obtenir un échantillon de largeur finale de 50mm. On place cet échantillon dans les mors d'un dynamomètre distants de 200mm. On procède à l'essai de traction jusqu'à la rupture de l'échantillon à la vitesse de 100mm/mn. On répète ainsi le test pour 5 échantillons. Les conditions de conditionnement, d'hygrométrie et de température sont définies dans la norme EN ISO 13934 - 1.

On a ainsi déterminé un allongement à la pose de 40% pour la bande selon l'exemple 6 et de 70% pour l'exemple 3 de l'invention de manière à appliquer à la pose une pression maximale de l'ordre de 50 à 70 mm de mercure. Pour poser la bande de façon appropriée, on a étalonné les bandes à l'aide d'un pochoir comme décrit dans la demande de brevet WO 2007/113430 page 13, ligne 18 à page 14, ligne 6.

Les résultats obtenus pour la bande obtenue selon les exemples de l'invention et le système de contention bicouches commercialisé par la société Laboratoires URGO sous la dénomination K2 taille 18-25 cm sont rassemblés dans les tableaux 1 ci-après.

La valeur «Pression Max à T0» correspond à la première pression de travail enregistrée immédiatement après la pose, et «Delta à T0>> correspond au différentiel de pression entre la première pression de travail et la première pression de repos enregistrées immédiatement après la pose. Les valeurs «Pression Max à T24» et «Delta à T24» correspondent aux mesures enregistrées 24 heures après la pose, mesurées en mm de mercure. Puis on a calculé la différence entre T0 et T24 heures «Delta (T0 - T24h)».

Tableau 1

| Exemple 6 | Exemple 3 | K2 (Laboratoires URGO) | Mesure effectuée |
|---|---|---|---|
| 40% | 70% | 55% + 50% | Allongement à la pose |
| 69 | 61 | 44 | Pression max à T0 |
| 28 | 26 | 19 | Delta à T0 |
| 51 | 38 | 35 | Pression max à T24 |
| 25 | 23 | 17 | Delta à T24 |
| +3 | +3 | +2 | Delta (TO-T24) |

Ce tableau montre que l'on obtient des résultats en terme de pressions appliquées à 24 heures et de différentiel de pression à 24 heures à la fois pour le système bi- couches K2 et les mono bandes selon l'invention qui sont compris dans les fourchettes visées à savoir un pression maximale à 24 heures comprises entre 35 et 50 mm de mercure et un différentiel de pression à 24 heures entre 15 et 25 mm de mercure. Les valeurs de différentiels de pression à 24 heures qui sont importantes pour l'efficacité du traitement sont mêmes supérieures pour les mono bandes selon l'invention, à savoir de 23 à 25 mm de mercure contre 17 mm de mercure pour le système bicouches K2. On constate aussi que pour les produits des exemples 3 et 6 et le produit K2, qui sont tous des allongements courts ce différentiel de pression varie peu au cours du temps puisque la variation est de +3 pour les tricots selon l'invention et + 2 pour le système bicouches K2. On obtient des résultats similaires que le fil d'entretoise soit un mono ou un multi filaments.

En conclusion les bandes selon l'invention permettent d'obtenir des propriétés thérapeutiques équivalentes à celles du produit K2 voire même supérieures, et de conserver ces dernières au cours du temps et ceci avec une seule bande et sans ajout de latex ou d'adhésif.

De la même façon on a comparé sur le test in vivo décrit ci-après les exemples 1 à 6, et le produit K2 pour évaluer le relâchement intrinsèque des bandes au cours du temps.

Le mode opératoire de ce test in vivo est le suivant.

On enroule les bandes autour de la jambe selon les préconisations décrites dans la notice du système bicouches K2.

Pour rappel, cette notice préconise la méthode d'application suivante :

1) Tenir le pied à 90°, orteils vers le haut. Appliquer KTECH à la base des orteils en faisant deux tous d'ancrage, en s'assurant que la face ouatée soit en contact direct avec la peau et que l'indicateur de pression soit situé sur le côté supérieur de la bande. Continuer en faisant un motif en « 8 » autour de la cheville, sans appliquer une tension excessive sur le pied et en recouvrant bien le talon.

2) Remonter jusqu'au genou en effectuant des spirales et en étirant la bande de manière appropriée : l'indicateur de pression imprimé sur les bandes doit former un cercle. Pour obtenir un recouvrement correct, l'indicateur de pression doit être tout juste recouvert (recouvrement de 50%). Finir 2cm sous le genou et couper l'excédent de bande. Fixer à l'aide d'un sparadrap.

3) Appliquer KPRESS sur KTECH selon la même technique en commençant un doigt au-dessus de KTECH et en finissant un doigt sous KTECH afin que seule KTECH soit en contact direct avec la peau. Une fois appliquée, appuyer doucement sur le bandage avec les mains pour assurer une bonne tenue du système.

On comprend bien que cette dernière étape 3) n'est pas nécessaire pour une bande de contention selon l'invention.

Pour les exemples selon l'invention on a déterminé comme précédemment pour le test in vitro un allongement à la pose pour obtenir une pression maximale à T = 0 comprise entre 50 et 70 mm de mercure et on a étalonné de la même façon les tricots en conséquence.

On enroule les bandes autour du pied, du talon et le long de la jambe jusqu'au genou avec un recouvrement d'une couche sur l'autre de 50%. La dernière spire est fixée sur elle-même à l'aide d'une attache métallique, d'un sparadrap ou de préférence avec 2 parties mâles avec crochets de bandes velcro. Si on souhaite vérifier la pression appliquée par la bande on peut disposer en un point B1, correspondant à la zone où le tendon d'Achille se transforme en muscle du mollet, soit de manière générale 10 à 15 cm environ au-dessus de la malléole, un capteur de mesure de pression d'interface comme par exemple le capteur référencée KKH-01 de la société KIKUHIME .On trace à l'aide d'un feutre fin et non effaçable un trait vertical sur au moins 3 spires, sur l'axe de la crête tibiale, à partir de la dernière spire enroulée.

Cette marque sert de référence pour évaluer à l'aide d'un réglet gradué au mm, le décalage horizontal du trait au terme de la durée du test. Lors des mouvements, ce trait perd de son caractère rectiligne et se présente en échelons d'autant plus décalés que les glissements spires sur spires sont importants. Si le glissement spire sur spire est très faible ou inexistant, le trait vertical reste intègre ou varie très peu principalement sur la première spire qui se situe sous la dernière spire enroulée.

Ce décalage du trait vertical est représentatif du relâchement de la bande et illustre son glissement potentiel au cours du temps.

Ce test a été réalisé durant 6 heures sur toujours la même personne. Cette personne porte sur une jambe une bande selon les exemples de 10 cm de largeur et 2,6 m de longueur, (face filet au contact de la peau si la bande possède une face filet), étalonnée en conséquence et sur l'autre jambe soit le système bicouches K2 soit une autre bande selon l'invention.

On mesure au bout des 6 heures le décalage du trait vertical sur les 4 premières spires.

Les résultats sont les suivants :

Système bicouches K2 : aucun décalage du trait sur aucune spire.

Un tel résultat est cohérent, du fait de la « cohésivation » de la bande qui bloque le glissement des spires les unes sur les autres.

Bande de contention selon les exemples 1 à 6 :

Les résultats obtenus pour ces 6 exemples sont rassemblés dans le tableau 2.

| exemple | contrainte | conformabilité | Spire 1 | Spire 2 | Spire 3 | Spire 4 | pose |
|---------|-----------|----------------|---------|---------|---------|---------|------|
| 1 | 2227 | 63,6 | 1 | 0 | 0 | 0 | 60% |
| 2 | 3667 | 70,3 | 1 | 0 | 0 | 0 | 85% |
| 3 | 2217 | 63,3 | 1 | 0 | 0 | 0 | 70% |
| 4 | 2207 | 67,1 | 15 | 6 | 4 | 4 | 70% |
| 5 | 1707 | 67,4 | 22 | 7 | 2 | 0 | 65% |
| 6 | 3080 | 66,2 | 4 | 0 | 0 | 0 | 40% |

Ce tableau illustre les caractéristiques essentielles que doit posséder un tricot 3D dont le fil d'entretoise est un multi filaments à savoir posséder une contrainte de cisaillement supérieure ou égale à 2800 Pa et/ou une conformabilité inférieure ou égale à 65mm.

Pour les produits des exemples 1, 2 et 3 qui possèdent au moins une de ces caractéristiques on ne constate au bout de 6 heures aucun décalage du trait sur les spires 2, 3 et 4 et un léger décalage de l'ordre de 1 mm sur la première spire qui se situe sous la dernière spire enroulée.

Cette valeur de 1 mm est négligeable. On considère qu'une valeur moyenne sur plusieurs personnes de 4 mm sur n'est pas représentative et représente les aléas de mesures liés à la variation des mollets des testeurs, la reproductibilité de la pose et les variabilités de fabrication des bandes.

C'est le résultat que l'on obtient avec l'exemple 6 qui a été testé sur 6 personnes différentes et qui est un mono filament.

L'importance de la présence d'au moins une de ces caractéristiques pour un tricot 3D, dont le fil d'entretoise est un multi filaments, est particulièrement mis en évidence quand on compare les produits entre eux.

Ainsi les exemples 3 et 4 ont des contraintes de cisaillement très proches de l'ordre de 2200 mais l'exemple 3 qui présente une conformabilité de 63,3 donc inférieure à 65 ne possède quasi aucun décalage spire sur spire alors que l'exemple 4 qui présente une conformabilité de 67,1 donc supérieure à 65 possède au bout de 6 heures un décalage sur les 4 spires et de 15mm sur la première spire.

Donc la conformabilité permet de compenser une contrainte de cisaillement trop faible. On retrouve ce résultat avec l'exemple 1 pour lequel la contrainte est de 2227 Pa mais la conformabilité de 63,6mm.

A l'inverse dans l'exemple 2 la contrainte est supérieure à 2800 Pa soit 3667 Pa et on observe aucun décalage spire sur spire bien que la conformabilité soit de 70,3mm.

Enfin l'exemple 5, qui ne présente aucune de ces caractéristiques, possède un décalage sur les 3 premières spires et de 22mm sur la première spire. Au global on retrouve les mêmes conclusions que le tricot possède une face ajourée (exemples 1, 2 et 5) ou 2 faces pleines (exemples 3 et 4).

En conclusion, même si les causes ne sont pas connues, un tricot 3D, dont le fil d'entretoise est un multi filaments, possède un comportement équivalent à celui, dans lequel le fil d'entretoise est un mono filament, s'il présente comme caractéristique essentielle une contrainte de cisaillement supérieure ou égale à 2800 Pa.

Si sa contrainte de cisaillement est inférieure à cette valeur s'il présente une conformabilité inférieure ou égale à 65mm cette caractéristique essentielle permet de compenser la trop faible valeur de contrainte de cisaillement.

On peut donc considérer qu'une bande selon l'invention qui présente au moins une de ces 2 caractéristiques possède une résistance au glissement spire sur spire équivalente à celle d'un système cohésivé ou à celui d'un tricot 3D dont le fil d'entretoise est un mono filament.

Ce test montre qu'en termes de tenue ces produits sont équivalents.

L'ensemble de ces tests in vivo et in vitro démontrent que l'on a bien obtenu avec une seule monobande un dispositif de contention qui présente les bonnes propriétés thérapeutiques et qui ne glisse pas au cours du temps sans l'ajout de substances supplémentaires sur cette dernière.

## Revendications

1. Bande de contention qui se présente sous la forme d'un tricot obtenu par la technologie maille jetée à base de fils synthétiques qui est constitué de 2 surfaces textiles dont la structure textile est identique ou différente, reliées entre elles par des fils d'entretoises, chaque surface comportant des fils élastiques, dans lequel lesdits fils d'entretoise sont des multi filaments,
le tricot présentant un allongement longitudinal mesuré selon la norme EN 14704 -1 compris entre 30 et 160%,
ledit tricot étant **caractérisé en ce qu'**il présente une contrainte de seuil de cisaillement supérieure ou égale à 2800 Pa et/ou une conformabilité inférieure ou égale à 65mm.

2. Bande de contention selon la revendication 1, dans laquelle le fil d'entretoise est un multi filament présentant un titre compris entre 20 et 80 dtex.

3. Bande de contention selon la revendication 2, dans lequel le fil d'entretoise présentant un titre compris entre 40 et 80 dtex.

4. Bande de contention selon l'une des revendications précédentes, dans laquelle ledit tricot présente une face qui a une structure textile parmi la liste ci-après,

   - charmeuse ;
   - demi-simple à mailles ouvertes ou fermées ;
   - atlas sous un ou plusieurs rangs ;
   - chainette à mailles ouvertes, fermées, ou à alternance de mailles fermées et ouvertes ;

   ladite face étant opposée à la face adaptée pour être mise en contact avec la peau qui a une structure textile qui est un filet, présentant une structure textile ajourée.

5. Bande de contention selon l'une des revendications précédentes dans laquelle le tricot présente une épaisseur comprise entre 1 et 2 mm.

6. Bande de contention selon la revendication 5, dans laquelle le tricot présente une épaisseur comprise entre 1 et 1,5 mm.

7. Bande de contention selon l'une des revendications précédentes, dans laquelle le tricot présente un écart entre les faces compris entre 0,4 et 1,5mm.

8. Bande de contention selon la revendication 7, dans laquelle le tricot présente un écart entre les faces compris entre 0,5 et 1,1mm.

9. Bande de contention selon l'une des revendications précédentes dans laquelle le tricot présente un grammage compris entre 160 et 370 g/m$^2$.

10. Bande de contention selon la revendication 8, dans laquelle le tricot présente un grammage compris entre 160 et 300 g/m$^2$.

11. Bande de contention selon l'une des revendications précédentes, dans laquelle le tricot présente un allongement longitudinal tel que défini dans la norme EN 14704-1 compris entre 50 et 120%.

12. Bande de contention selon l'une des revendications précédentes dans laquelle le tricot est fabriqué en utilisant une seule barre pour le fil d'entretoise qui relie les 2 faces textiles.

13. Bande de contention selon l'une des revendications précédentes dans laquelle le fil élastique présente un titre compris entre 40 et 80 dtex.

14. Bande de contention selon l'une des revendications précédentes dans laquelle les surfaces textiles comprennent des fils thermoplastiques présentant des titres de 40 à 90 dtex.

15. Kit qui comprend une ou plusieurs bandes de contention selon l'une des revendications précédentes et un ou plusieurs pansements adaptés pour être disposés sur une plaie préalablement à la bande de contention.


**Patentansprüche**

1. Kompressionsbandage, die die Form eines Gewirks aufweist, das durch die Kettengewirktechnologie auf Basis von synthetischen Fäden erhalten wird und das aus 2 Textiloberflächen besteht, deren Textilstruktur identisch oder unterschiedlich ist und die untereinander durch Querfäden verbunden sind, wobei jede Oberfläche elastische Fäden beinhaltet, wobei die Querfäden Multifilamente sind,
wobei das Gewirk eine gemäß der Norm EN 14704-1 gemessene Längsdehnung von zwischen 30 und 160 % aufweist,
wobei das Gewirk **dadurch gekennzeichnet ist, dass** es eine Grenzscherspannung von größer oder gleich 2800 Pa und/oder eine Formanpassung von kleiner oder gleich 65 mm aufweist.

2. Kompressionsbandage nach Anspruch 1, wobei der Querfaden ein Multifilament ist, das einen Titer von zwischen 20 und 80 dtex aufweist.

3. Kompressionsbandage nach Anspruch 2, wobei der Querfaden einen Titer von zwischen 40 und 80 dtex aufweist.

4. Kompressionsbandage nach einem der vorhergehenden Ansprüche, wobei das Gewirk eine Seite aufweist, die eine Textilstruktur von der nachfolgenden Liste aufweist,

   - Charmeuse,
   - offene oder geschlossene Halbmasche,
   - Atlas unter einer oder mehreren Reihen,
   - Franse mit offener Legung, geschlossener Legung oder mit Wechsel von geschlossener und offener Legung,

   wobei die Seite der Seite entgegengesetzt ist, die dafür geeignet ist, mit der Haut in Kontakt gebracht zu werden, die eine Textilstruktur aufweist, die ein Netz ist, das eine durchbrochene Textilstruktur aufweist.

5. Kompressionsbandage nach einem der vorhergehenden Ansprüche, wobei das Gewirk eine Dicke von zwischen 1 und 2 mm aufweist.

**6.** Kompressionsbandage nach Anspruch 5, wobei das Gewirk eine Dicke von zwischen 1 und 1,5 mm aufweist.

**7.** Kompressionsbandage nach einem der vorhergehenden Ansprüche, wobei das Gewirk einen Abstand von zwischen 0,4 und 1,5 mm zwischen den Seiten aufweist.

**8.** Kompressionsbandage nach Anspruch 7, wobei das Gewirk einen Abstand von zwischen 0,5 und 1,1 mm zwischen den Seiten aufweist.

**9.** Kompressionsbandage nach einem der vorhergehenden Ansprüche, wobei das Gewirk ein Flächengewicht von zwischen 160 und 370 g/m$^2$ aufweist

**10.** Kompressionsbandage nach Anspruch 8, wobei das Gewirk ein Flächengewicht von zwischen 160 und 300 g/m$^2$ aufweist.

**11.** Kompressionsbandage nach einem der vorhergehenden Ansprüche, wobei das Gewirk eine gemäß der Norm EN 14704-1 definierte Längsdehnung von zwischen 50 und 120 % aufweist.

**12.** Kompressionsbandage nach einem der vorhergehenden Ansprüche, wobei das Gewirk unter Verwendung einer einzigen Barre für den Querfaden hergestellt ist, der die 2 Textilseiten verbindet.

**13.** Kompressionsbandage nach einem der vorhergehenden Ansprüche, wobei der elastische Faden einen Titer von zwischen 40 und 80 dtex aufweist.

**14.** Kompressionsbandage nach einem der vorhergehenden Ansprüche, wobei die Textiloberflächen thermoplastische Fäden umfassen, die Titer von 40 bis 90 dtex aufweisen.

**15.** Kit, das eine oder mehrere Kompressionsbandagen nach einem der vorhergehenden Ansprüche und einen oder mehrere Verbände umfasst, der/die dafür geeignet ist/sind, vor der Kompressionsbandage auf einer Wunde angeordnet zu werden.

**Claims**

**1.** Compression bandage in the form of a knit obtained with warp knit technology, formed of synthetic yarns and composed of 2 textile surfaces having the same or different textile structure, linked together by spacer threads, each surface comprising elastic yarns, wherein said spacer threads are multifilaments,,
the knit having a longitudinal elongation measured as per standard EN 14704 -1 of between 30 and 160 %;
the knit having a threshold shear stress equal to or higher than 2800 Pa and/or conformability equal to or lower than 65 mm, said knit being **characterized in that** it has a threshold shear stress equal to or higher than 2800 Pa, and/or conformability equal to or lower than 65 mm.

**2.** The compression bandage according to claim 1, wherein the spacer thread is a multifilament having a grade of between 20 and 80 dtex.

**3.** The compression bandage according to claim 2, wherein the spacer thread has a grade of between 40 and 80 dtex.

**4.** The compression bandage according to one of the preceding claims, wherein said knit has one surface having a textile structure from among the following list:

- locknit;
- open or closed loop single tricot;
- atlas under one or more rows;
- open or closed loop pillar stitch, or alternating closed and open loops;

said surface lying opposite a surface adapted to be placed in contact with the skin and having a net textile structure with openwork textile structure.

**5.** The compression bandage according to one of the preceding claims, wherein the knit has a thickness of between 1 and 2 mm.

6. The compression bandage according to claim 5, wherein the knit has a thickness of between 1 and 1.5 mm.

7. The compression bandage according to one of the preceding claims, wherein the knit has a spacing between the surfaces of between 0.4 and 1.5 mm.

8. The compression bandage according to claim 7, wherein the knit has a spacing between the surfaces of between 0.5 and 1.1 mm.

9. The compression bandage according to one of the preceding claims, wherein the knit has a gram weight of between 160 and 370 g/m$^2$.

10. The compression bandage according to claim 8, wherein the knit has a gram weight of between 160 and 300 g/m$^2$.

11. The compression bandage according to one of the preceding claims, wherein the knit has longitudinal elongation such as defined in standard EN 14704-1 of between 50 and 120 %.

12. The compression bandage according to one of the preceding claims, wherein the knit is produced using a single bar for the spacer thread which links together the 2 textile surfaces.

13. The compression bandage according to one of the preceding claims, wherein the grade of the elastic yarn is between 40 and 80 dtex.

14. The compression bandage according to one of the preceding claims, wherein the textile surfaces comprise thermoplastic yarns having grades of 40 to 90 dtex.

15. Kit comprising one or more compression bandages according to one of the preceding claims and one or more dressings adapted to be placed over a wound prior to the compression bandage.

FIG.1

FIG.2

FIG.3

FIG.4

FIG.5

**RÉFÉRENCES CITÉES DANS LA DESCRIPTION**

**Documents brevets cités dans la description**

- WO 9516416 A
- WO 200971894 A
- EP 1052319 A
- GB 2473321 A
- WO 2007113430 A